Europäisches Patentamt

European Patent Office

Office européen des brevets

(19)

(11) Numéro de publication : **0 420 729 B1**

(12)
# FASCICULE DE BREVET EUROPEEN

(45) Date de publication du fascicule du brevet :
**28.04.93 Bulletin 93/17**

(51) Int. Cl.$^5$ : **A61K 35/78,** C12N 5/00,
A23L 1/221

(21) Numéro de dépôt : **90402603.6**

(22) Date de dépôt : **20.09.90**

(54) **Procédé de préparation de suspensions homogènes de plantes et suspensions homogènes ainsi obtenues.**

(30) Priorité : **28.09.89 FR 8912689**

(43) Date de publication de la demande :
**03.04.91 Bulletin 91/14**

(45) Mention de la délivrance du brevet :
**28.04.93 Bulletin 93/17**

(84) Etats contractants désignés :
**AT BE CH DE DK ES FR GB GR IT LI LU NL SE**

(56) Documents cités :
**EP-A- 0 085 589**
**FR-A- 2 608 923**

(73) Titulaire : **LABORATOIRES ARDEVAL**
**29 rue Marcel Cachin**
**F-94204 Ivry sur Seine (FR)**

(72) Inventeur : **Derrieu, Guy**
**Le Riviera Parc, 33 Chemin du Lautin**
**F-06800 Cagnes Sur Mer (FR)**
Inventeur : **Le Pennec, Dominique**
**Villa La Tarasque, Chemin des Espeiroures**
**F-06510 Gattières (FR)**

(74) Mandataire : **Ores, Irène et al**
**CABINET ORES 6, Avenue de Messine**
**F-75008 Paris (FR)**

EP 0 420 729 B1

## Description

La présente invention est relative à un procédé de préparation de suspensions homogènes de plantes entières et/ou de parties de plantes fraîches ou sèches ainsi qu'aux suspensions homogènes obtenues par ce procédé.

On entend, au sens de la présente invention, par partie de plante, les racines, tiges, feuilles, sommités fleuries, fleurs, fruits ou graines.

Il est connu que les plantes constituent de véritables réservoirs de principes actifs et sont toujours très utilisées soit telles quelles, sous forme sèche notamment, soit sous forme d'extraits, soit sous forme de suspensions dans de nombreuses préparations et notamment des préparations à usage pharmaceutique, vétérinaire, cosmétologique ou alimentaire.

Parmi les formes liquides à base de plantes, on peut distinguer essentiellement les extraits et les suspensions.

Les extraits présentent un certain nombre d'inconvénients :
- ils ne permettent pas de contenir la totalité des principes actifs ; en effet, l'action du solvant, selon sa nature, n'entraîne que les composés qu'il solubilise ;
- le solvant d'extraction n'est pas inerte, il peut entraîner des dégradations, surtout lorsque les opérations d'extraction font intervenir la chaleur (par exemple macération à chaud, distillation ou autres procédés contraignants) ;
- de plus, pour avoir la meilleure extraction il est préférable de diviser mécaniquement les végétaux, mais cette opération entraîne une élévation de température néfaste aussi bien pour les matériaux frais que secs, car on augmente les surfaces d'oxydation, d'évaporation, phénomènes intensifiés par l'élévation de chaleur. Ces inconvénients croissent avec la finesse des particules. Afin de lutter contre ce dernier inconvénient on limite la division par un broyage grossier, qui diminue l'efficacité extractive du solvant.

Un Brevet français n° 2 608 923, décrit des procédés d'obtention de nouveaux extraits végétaux liquides de plantes fraîches, mais lesdits procédés ne permettent pas de contenir l'intégralité des principes actifs de la plante, pour les raisons invoquées ci-dessus.

En ce qui concerne les suspensions, leur préparation comporte au moins deux grands écueils :
- le premier écueil est constitué par le broyage lui-même : s'il est pratiqué sans précautions, il a pour effet immédiat de mettre en contact des sites qui, à l'état normal, étaient géographiquement séparés et, de ce fait, favoriser l'action d'enzymes sur des substrats ;
- un autre écueil est constitué par divers processus physiques et chimiques mis en oeuvre (tels que température, pH, solvants, etc...) qui tous ont une influence plus ou moins néfaste sur les principes actifs et l'équipement enzymatique des plantes.

La lyophilisation par exemple, qui permet un séchage rapide à basse température, outre qu'elle constitue une technique coûteuse, provoque la perte par évaporation des divers principes volatils tels que les huiles essentielles, certaines amines et divers arômes. Elle provoque même certaines altérations dues à la phase ultime du séchage fait à une température voisine de 20°C.

Il y a également des risques sérieux de dégradation par l'extraction aux divers solvants, très largement utilisée et qui fait appel à la solubilisation des substances contenues dans les plantes. En outre, cette solubilisation n'est pas complète, même si l'on fait appel à toute une série de solvants successifs. L'évaporation du solvant (ou même l'extraction elle-même), se fait parfois à chaud et est donc dangereuse pour les divers principes actifs. De plus, les résidus des divers solvants s'avèrent souvent toxiques.

Dans le Brevet européen 85 589, un procédé de préparation de suspensions de cellules fraîches stabilisées, qui préserve la quasi-totalité des principes actifs de la cellule, est décrit. Ce procédé comporte notamment un broyage à froid de manière à obtenir une granulométrie inférieure à environ 100 μm, puis deux micronisations successives effectuées en présence d'un solvant miscible à l'eau et à froid.

Un tel procédé a l'avantage d'éviter les écueils précisés ci-dessus mais ne permet toutefois d'obtenir que les principes actifs solubles dans le solvant utilisé ; or dans certains cas, des principes non solubles présentent des propriétés intéressantes soit du point de vue thérapeutique, soit du point de vue cosmétologique, par exemple. De plus, un tel procédé n'élimine pas les éléments structuraux des végétaux (la cellulose, l'hemi-cellulose, la lignine, le suber), qui perturbent la biodisponibilité des principes actifs et de leur environnement et, entre autres, provoque des fermentations intestinales lors d'ingestion.

La présente invention s'est par conséquent donné pour but de pourvoir à un procédé de préparation de suspensions homogènes de plantes entières et/ou de parties de plantes, qui répond mieux aux nécessités de la pratique que les procédés visant au même but et antérieurement connus notamment en ce qu'il permet d'obtenir tous les principes actifs de la plante tout en éliminant les éléments structuraux des végétaux.

La présente invention a pour objet un procédé de préparation de suspensions homogènes de plantes fraî-

2

ches ou sèches, comportant des étapes de nettoyage poussé de la plante ou partie de plante et/ou de séchage, de cryobroyage à une température inférieure à 0°C jusqu'à l'obtention d'une granulométrie inférieure à environ 100 μm, lequel procédé est caractérisé :

1) en ce qu'après un trempage de quelques heures à plus de 72 heures des particules dans au moins un solvant apte à extraire le/les principes actifs de la plante ou partie de plante à traiter, solubles dans ledit/lesdits solvants, la phase liquide et la phase solide de la suspension obtenue sont soumises à une séparation de phases ;

2) en ce que la phase solide est exprimée par pressage à froid ;

3) puis en ce que la phase liquide obtenue en 1) et le pressat obtenu en 2) sont mélangés et constituent une suspension homogène et intégrale de plantes et/ou parties de plantes.

Au sens de l'invention, "intégrale" signifie que tous les principes actifs (solubles ou insolubles dans le solvant) présents dans la plante ou la partie de plante sont présents dans la suspension homogène.

Un tel procédé, conforme à l'invention, présente les avantages suivants :

- il permet de traiter les plantes à froid, d'où une plus grande stabilité des principes ;
- il permet d'avoir une extraction totale aussi bien des corps polaires qu'apolaires, en raison de l'étape de pressage à froid ; en effet, l'extraction des principes actifs solubles et insolubles dans le solvant est réalisée et est obtenue par la combinaison solvant-pressage à froid. L'extraction des principes actifs ne dépend plus de la nature du solvant et on peut ainsi éviter l'emploi de solvants non recommandés pour des usages pharmaceutiques et même cosmétologiques ;
- il permet de traiter en même temps des parties différentes de la plante (par exemple racines et/ou tiges, feuilles, fleurs ou fruits) qui, nécessiteraient des procédés d'extraction et/ou des solvants différents pour obtenir les principes actifs intéressants ;
- il permet par le choix du solvant, de conserver les principes dans leur état initial avant traitement, de bloquer les enzymes sans les détruire et de stabiliser la préparation.

Les plantes sont de préférence des plantes à usage médicinal, tinctorial, aromatique, alimentaire ou cosmétologique.

Selon un autre mode de mise en oeuvre avantageux du procédé conforme à l'invention, le trempage de l'étape 1) est réalisé à l'aide d'au moins un solvant choisi dans le groupe qui comprend l'eau, les alcools comme l'éthanol, les polyalcools comme le propylène glycol, le glycérol, le sorbitol, les alcools de sucres, les maltodextrines, les cyclodextrines, les esters d'acides gras comme le myristate d'isopropyle, les corps gras comme les huiles minérales, végétales ou animales ou les tensio-actifs.

Dans le cas où le trempage est réalisé à l'aide de plusieurs solvants, ceux-ci sont miscibles entre eux ou rendus miscibles, notamment en présence d'agents tensio-actifs.

Selon une disposition avantageuse de ce mode de mise en oeuvre, le/les solvants sont au moins dans un rapport 1:1 par rapport au poids de la plante à traiter.

La quantité de solvant ajoutée doit être suffisante pour pouvoir imbiber la plante ; en effet, une imbibation correcte de la plante, permet lors du pressage, d'extraire tous les principes actifs insolubles.

La séparation de la phase liquide et de la phase solide est réalisée par une technique de séparation connue en soi, notamment par décantation, par centrifugation ou par filtration.

Selon un autre mode de mise en oeuvre avantageux du procédé conforme à l'invention, la pression exercée lors de l'étape 2) dépend de la plante ou partie de plante à traiter.

Conformément à l'invention, elle peut avantageusement être comprise entre 1 bar et 100 bars.

De plus hautes pressions pourraient, dans certains cas, également être appliquées.

La vitesse de montée en pression dépend également de la plante à traiter et est de l'ordre de quelques minutes.

Selon un autre mode de mise en oeuvre du procédé conforme à l'invention, le pressage est réalisé à l'aide d'une presse à compression radiale, la phase solide étant répartie en fines couches.

On obtient ainsi de très bons résultats, à savoir l'extraction de tous les principes actifs insolubles dans le/les solvants précédemment utilisés, sans échauffement. De plus, le cryobroyage du matériel végétal de départ offre une plus grande surface de contact au solvant, une meilleure hydratation et donc une meilleure expression.

La présente invention a également pour objet une suspension homogène intégrale de plantes entières et/ou de parties de plantes fraîches ou sèches obtenues au moyen du procédé conforme à l'invention.

Selon un mode de réalisation avantageux de ladite suspension homogène, celle-ci est concentrée de manière appropriée.

La présente invention a également pour objet une composition liquide, caractérisée en ce qu'elle comprend une suspension homogène intégrale conforme à l'invention associée à au moins l'un des agents suivants : arômes, édulcorants, épaississants, parfums, gélifiants, agents thixotropes, appétents.

Selon un mode de réalisation avantageux de ladite composition liquide, elle comprend une suspension homogène intégrale de plantes conforme à l'invention, au moins un arôme, au moins un édulcorant et au moins un agent thixotrope.

Ledit agent thixotrope est notamment adapté au solvant utilisé lors du trempage, de manière à obtenir une composition homogène et agréable à prendre.

La présente invention a également pour objet une composition solide, caractérisée en ce qu'elle comprend une suspension homogène intégrale de plante conforme à l'invention mise sous une forme solide.

Selon un mode de réalisation avantageux de ladite composition, la suspension homogène intégrale de plante est absorbée sur un support solide absorbant approprié.

On peut citer notamment comme support solide, et ce de manière non limitative, les cyclodextrines, les maltodextrines ou tout autre support absorbant.

Selon un autre mode de réalisation de ladite composition, la suspension homogène est solidifiée par une opération physique telle que dessiccation, évaporation, lyophilisation ou atomisation.

Les suspensions homogènes et compositions conformes à l'invention trouvent application dans le domaine pharmaceutique, cosmétologique ou alimentaire.

Outre les dispositions qui précédent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention dont ils ne constituent en aucune manière une limitation.

**EXEMPLE 1 : Préparation intégrale de Bardane (*Arctium majus* ou *Lappa major*).**

On prépare une suspension homogène comme pré cisé ci-après avec les constituants suivants :

partie utilisée : racines fraîches
quantité mise en oeuvre : équivalent à 10% de racines sèches (taux R)
solvants utilisés : éthanol-eau (30/70)

On détermine le taux R de matière sèche comme décrit dans le Brevet européen 85 589.

Les racines fraîches, aussitôt après la récolte, sont triées, lavées soigneusement sous un courant d'eau froide, égouttées sur un grillage en acier inoxydable puis plongées dans de l'azote liquide (-196°C).

On prélève 13,4 kg de racines fraîches congelées et on les place dans un broyeur à couteau, travaillant en continu, préalablement refroidi et maintenu à - 50°C par addition d'azote liquide.

On réalise un mélange éthanol-eau dans une cuve (30/70) et on refroidit l'ensemble à une température inférieure à 10°C.

On ajoute la plante cryobroyée, on agite en maintenant la température au voisinage de 10°C. On ajuste le volume à 100 litres avec de l'eau et/ou de l'éthanol, afin d'avoir un degré alcoolique voisin de 30 % (m/m).

Le mélange est conservé pendant 48 heures puis la phase liquide et la phase solide sont séparées par centrifugation à 1 000 t/min. Le pressage est réalisé, par exemple, comme suit, à titre d'exemple non limitatif :

La phase solide est récupérée et est répartie en couche mince, de l'ordre de 1 à 2 cm sur une toile de pressage (10) qui est déroulée en continu et qui, après la répartition de la phase solide, est embobinée sur une grosse bobine (20) ; ladite grosse bobine (20) est ensuite insérée dans une virole (30). La pression est alors montée à 50 bars en 5 minutes et maintenue à cette pression pendant un quart d'heure. Le liquide exprimé s'écoule à travers la toile de pressage (10) et est récupéré.

Ce procédé de pressage a l'avantage d'exercer une pression homogène sur l'ensemble de la phase solide.

La phase liquide et le pressat obtenu sont mélangés et homogénéisés.

On obtient ainsi une suspension homogène intégrale et homogène de Bardane, d'aspect brunâtre et à odeur caractéristique et donc le degré alcoolique est de 29,7°.

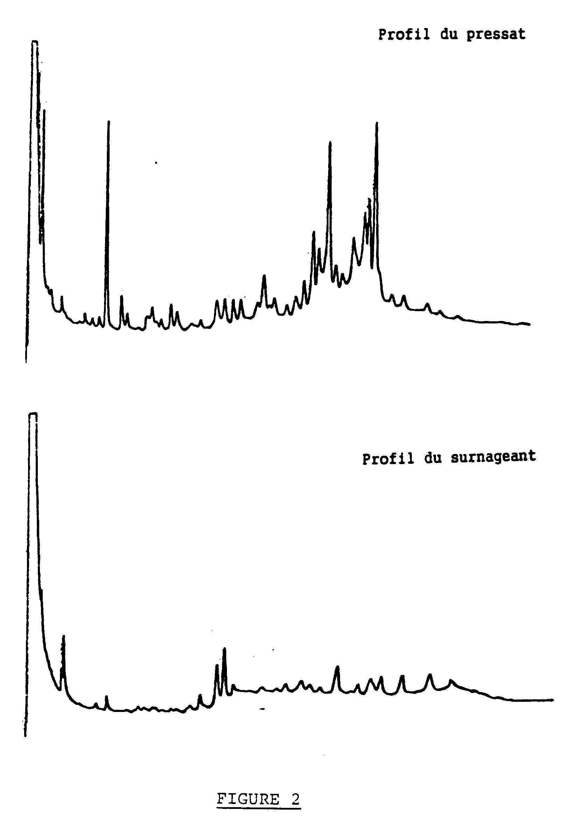

La contribution particulière du pressat dans la composition finale du mélange a été mise en évidence par chromatographie en phase gazeuse. L'étude du profil chromatographique d'un extrait chlorométhylique de pressat révèle la présence de nombreux composés, apparentés aux acides gras, absents dans le profil chromatographique d'un extrait de la phase liquide. Ceci s'explique par la nature du solvant utilisé, seuls les composés solubles en milieu hydroalcoolique, c'est-à-dire les polaires, sont extraits. L'opération physique de pressage permet de collecter les apolaires mais aussi le solde du solvant hydroalcoolique imbibant les particules végétales. Ainsi la réunion de la phase liquide et du pressat donne lieu à une suspension homogène contenant l'intégralité active de la plante, ce que fait clairement apparaître le chromatogramme qui fait l'objet de la figure 2 annexée, qui représente respectivement le profil du pressat et le profil du surnageant.

La chromatographie a été réalisée en utilisant une colonne semi-capillaire DB 5 de 15 m x 0,53 μm avec comme gaz vecteur de l'hydrogène, une température de 300°C à l'injecteur et de FID 300°C au détecteur. La température a été programmée à 100°C à 250°C par 4°C/min.

Une étude analytique de la plante fraîche et de la suspension homogène a été réalisée. Les résultats sont exprimés par rapport aux racines séchées pour la plante fraîche ou l'équivalent en racines séchées pour la suspension homogène.

|  | plante fraîche | pseudo-solution |
|---|---|---|
| **ELEMENTS MINERAUX** | | |
| Azote | 1,22 % | 1,4 % |
| Potassium | 1,2 % | 1,3 % |
| Calcium | 0,32 % | 0,2 % |
| Silicium | 0,27 % | 0,1 % |
| Phosphore | 0,21 % | 0,16 % |
| Magnésium | 0,11 % | 0,12 % |
| **PROTIDES** | | |
| totaux | 1,21 % | 1,6 % |
| ac. aminés libres | 0,185 % | 0,3 % |
| ac. aminés après hydrolyse | 0,94 % | 1,2 % |
| **SUCRES** | | |
| totaux | 8,35 % | 9 % |
| inuline | 8 % | 8,3 % |
| glucose | 0,165 % | 0,3 % |
| saccharose | 0,165 % | 0,2 % |
| **DIVERS** | | |
| lipides | traces | traces |
| vitamine E | 0,000015 % | 0,000012% |
| flavonoïdes | 0,07 % | 0,13% |
| stérols | 0,001 % | 0,0007% |
| tanins | présence | présence |
| saponosides | présence | présence |

On remarque la forte similitude entre les deux analyses avec un peu plus de composés polaires extraits dans la suspension homogène dû au choix du solvant.

**EXEMPLE 2 : Préparation intégrale de Mélilot (*Melilotus officinalis*).**

On prépare la suspension homogène selon le protocole de fabrication énoncé à l'Exemple 1, avec les constituants suivants :

partie utilisée : sommités fleuries
quantité mise en oeuvre : équivalent à 10% de plantes sèches (taux R)
solvants utilisés : éthanol-eau (30/70)
Contrôle du produit:
Aspect : liquide fluide marron clair à odeur caractéristique
degré alcoolique : 29,5°

Une étude analytique de la plante fraîche et de la suspension homogène a été réalisée. Les résultats sont exprimés par rapport à la plante séchée pour la plante fraîche ou l'équivalent en plante séchée pour la suspension homogène.

|  | plante fraîche | pseudo-solution |
|---|---|---|
| **ELEMENTS MINERAUX** | | |
| Azote | 2,8 % | 2,7 % |
| Potassium | 1,8 % | 1,9 % |
| Calcium | 1,43 % | 0,9 % |
| Phosphore | 0,24 % | 0,26 % |
| Magnésium | 0,22 % | 0,21 % |
| **PROTIDES** | | |
| totaux | 2,07 % | Ã 2,3 % |
| ac. aminés libres | 0,168 % | 0,24 % |
| ac. aminés après hydrolyse | 1,56 % | 2 % |
| **SUCRES** | | |
| totaux | 6,96 % | 7,3 % |
| glucose | 0,66 % | 0,72 % |
| lactose | traces | traces |
| saccharose | absence | absence |
| **DIVERS** | | |
| lipides | 0,148 % | 0,134 % |
| vitamine C | 0,00174 % | 0,0019% |
| vitamine K | 0,0001% | 0,00008% |
| flavonoïdes | 0,23 % | 0,38 % |
| stérols | 0,003 % | 0,0027% |
| coumarine | 0,25 % | 0,27 % |
| saponosides | présence | présence |
| allantoïne | 0,2 % | 0,2 % |

Mêmes remarques que pour l'exemple 1.

**EXEMPLE 3 : Préparation de suspension homogènes intégrales d'aubépine et de pissenlit.**

On prépare les deux suspensions selon le protocole de fabrication énoncé ci-dessus avec les plantes suivantes :

a) sommités fleuries d'aubépine (équivalent à 5 % de plantes sèches),

b) racines de pissenlit (équivalent à 5 % de plantes sèches).

On met en évidence le rôle du pressat par l'étude des profils chromatographiques des extraits chlorométhyliques.

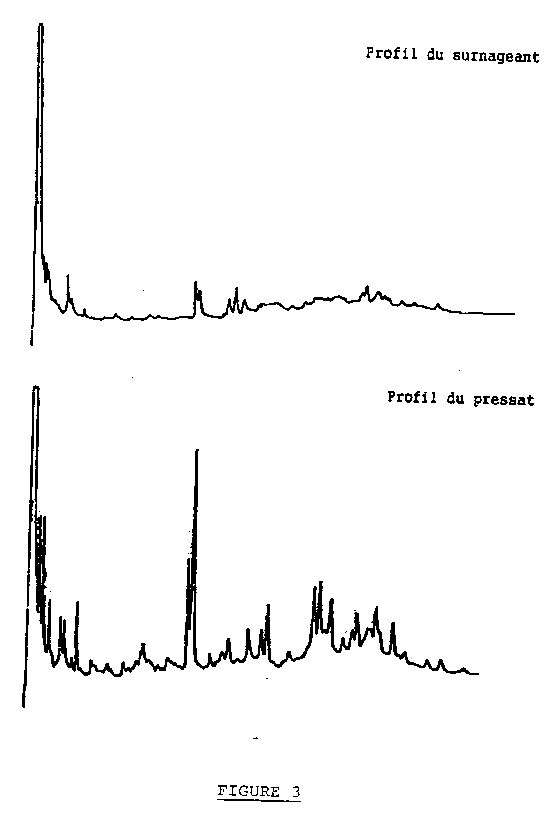

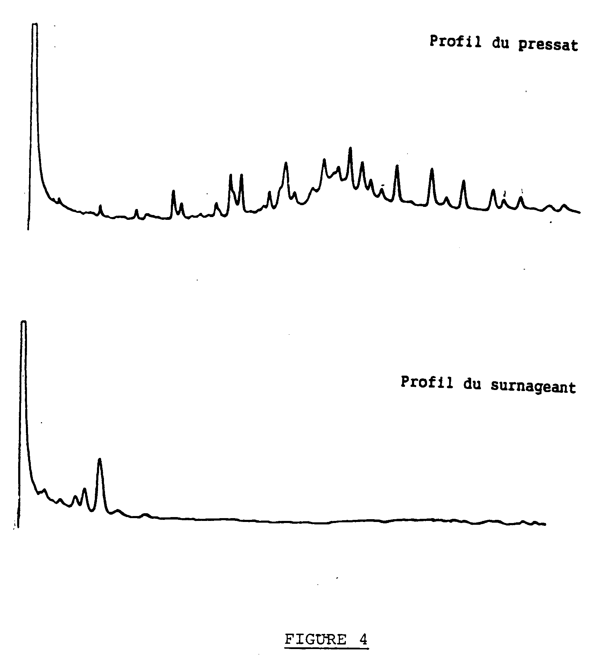

Les chromatogrammes représentés aux figures 3 et 4 représentent respectivement :

- figure 3 : le chromatogramme de l'aubépine avec le profil du surnageant et le profil du pressat obtenus dans les conditions indiquées pour la chromatographie à l'Exemple 1 ;

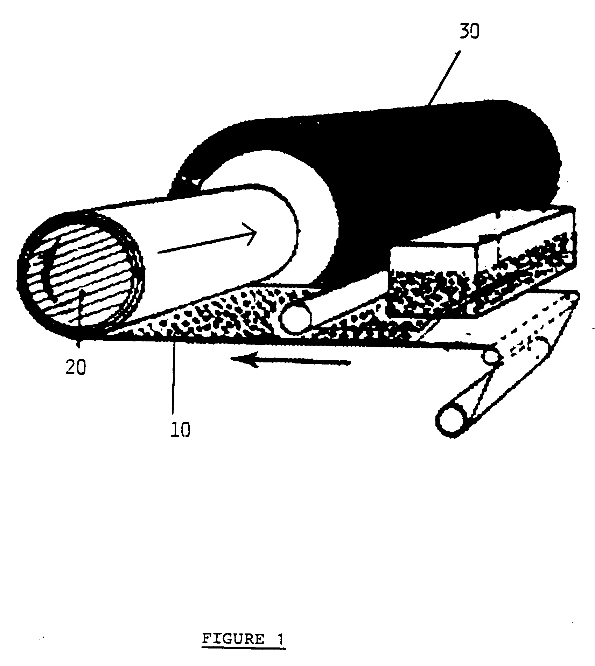

- figure 4 : le chromatogramme de la bardane avec le profil du pressat et le profil du surnageant également obtenus dans les mêmes conditions que celles décrites pour la chromatographie à la figure 1.

**Revendications**

**Revendications pour les Etats contractants suivants : AT, BE, CH, DE, DK, FR, GB, IT, LI, LU, NL, SE**

1. Procédé de préparation de suspensions homogènes de plantes fraîches ou sèches, comportant des étapes de nettoyage poussé de la plante ou partie de plante et/ou de séchage, de cryobroyage à une température inférieure à 0°C jusqu'à l'obtention d'une granulométrie inférieure à environ 100 µm, lequel procédé est caractérisé :

1) en ce qu'après un trempage de quelques heures à plus de 72 heures des particules dans au moins un solvant apte à extraire le/les principes actifs de la plante ou partie de plante à traiter, solubles dans ledit/lesdits solvants, la phase liquide et la phase solide de la suspension obtenue sont soumises à une séparation de phases ;

2) en ce que la phase solide est exprimée par pressage à froid ;

3) puis en ce que la phase liquide obtenue en 1) et le pressat obtenu en 2) sont mélangés et constituent une suspension homogène et intégrale de plantes et/ou parties de plantes.

2. Procédé selon la revendication 1, caractérisé en ce que le trempage de l'étape 1) est réalisé à l'aide d'au moins un solvant choisi dans le groupe qui comprend l'eau, les alcools comme l'éthanol, les polyalcools comme le propylène glycol, le glycérol, le sorbitol, les alcools de sucres, les maltodextrines, les cyclodextrines, les esters d'acides gras comme le myristate d'isopropyle, les corps gras comme les huiles minérales, végétales ou animales ou les tensio-actifs.

3. Procédé selon la revendication 2, caractérisé en ce que le/les solvants sont au moins dans un rapport 1:1 par rapport au poids de la plante à traiter.

4. Procédé selon l'une quelconque des revendications 1 à 3, caractérisé en ce que la pression exercée lors de l'étape 2) dépend de la plante ou partie de plante à traiter et est de préférence comprise entre 1 et 100 bars.

5. Procédé selon l'une quelconque des revendications 1 à 4, caractérisé en ce que le pressage est réalisé à l'aide d'une presse à compression radiale, la phase solide étant répartie en fines couches.

6. Composition liquide, caractérisée en ce qu'elle comprend une suspension homogène intégrale de plantes obtenue au moyen du procédé selon l'une quelconque des revendications 1 à 5, au moins un arôme, au moins un édulcorant et au moins un agent thixotrope.

7. Composition liquide selon la revendication 6, caractérisée en ce qu'elle comprend en outre au moins l'un des agents suivants : épaississants, parfums, gélifiants, appétents.

8. Composition solide, caractérisée en ce qu'elle comprend une suspension intégrale de plante obtenue au moyen du procédé selon l'une quelconque des revendications 1 à 5, mise sous une forme solide.

9. Composition selon la revendication 8, caractérisée en ce que la suspension homogène intégrale de plante est absorbée sur un support solide absorbant approprié.

10. Composition selon la revendication 8, caractérisée en ce que la suspension homogène est solidifiée par une opération physique telle que dessiccation, évaporation, lyophilisation ou atomisation.

11. Utilisation de la composition selon l'une quelconque des revendications 6 à 10, pour la préparation d'un médicament.

12. Utilisation des suspensions homogènes intégrales de plantes obtenues au moyen du procédé selon l'une quelconque des revendications 1 à 5, pour la préparation d'un médicament.

13. Application de la composition selon l'une quelconque des revendications 6 à 10 et des suspensions homogènes obtenues au moyen du procédé selon l'une quelconque des revendications 1 à 5, à l'usage cosmétologique ou alimentaire.

**Revendications pour les Etats contractants suivants : ES, GR**

1. Procédé de préparation de suspensions homogènes de plantes fraîches ou sèches, comportant des étapes de nettoyage poussé de la plante ou partie de plante et/ou de séchage, de cryobroyage à une température inférieure à 0°C jusqu'à l'obtention d'une granulométrie inférieure à environ 100 µm, lequel procédé est caractérisé :

1) en ce qu'après un trempage de quelques heures à plus de 72 heures des particules dans au moins un solvant apte à extraire le/les principes actifs de la plante ou partie de plante à traiter, solubles dans ledit/lesdits solvants, la phase liquide et la phase solide de la suspension obtenue sont soumises à une séparation de phases ;

2) en ce que la phase solide est exprimée par pressage à froid ;

3) puis en ce que la phase liquide obtenue en 1) et le pressat obtenu en 2) sont mélangés et constituent une suspension homogène et intégrale de plantes et/ou parties de plantes.

2. Procédé selon la revendication 1, caractérisé en ce que le trempage de l'étape 1) est réalisé à l'aide d'au moins un solvant choisi dans le groupe qui comprend l'eau, les alcools comme l'éthanol, les polyalcools comme le propylène glycol, le glycérol, le sorbitol, les alcools de sucres, les maltodextrines, les cyclodextrines, les esters d'acides gras comme le myristate d'isopropyle, les corps gras comme les huiles minérales, végétales ou animales ou les tensio-actifs.

3. Procédé selon la revendication 2, caractérisé en ce que le/les solvants sont au moins dans un rapport 1:1 par rapport au poids de la plante à traiter.

4. Procédé selon l'une quelconque des revendications 1 à 3, caractérisé en ce que la pression exercée lors de l'étape 2) dépend de la plante ou partie de plante à traiter et est de préférence comprise entre 1 et 100 bars.

5. Procédé selon l'une quelconque des revendications 1 à 4, caractérisé en ce que le pressage est réalisé à l'aide d'une presse à compression radiale, la phase solide étant répartie en fines couches.

6. Procédé de préparation d'une composition liquide, caractérisé en ce qu'on mélange une suspension homogène intégrale de plantes obtenue au moyen du procédé selon l'une quelconque des revendications 1 à 5, au moins un arôme, au moins un édulcorant et au moins un agent thixotrope.

7. Procédé selon la revendication 6, caractérisé en ce qu'on ajoute en outre au moins l'un des agents suivants : épaississants, parfums, gélifiants, appétents.

8. Procédé de préparation d'une composition solide, caractérisé en ce qu'on met sous une forme solide, une suspension intégrale de plante obtenue au moyen du procédé selon l'une quelconque des revendications 1 à 5.

9. Procédé de préparation selon la revendication 8, caractérisé en ce qu'on absorbe la suspension homogène intégrale de plante sur un support solide absorbant approprié.

10. Procédé de préparation selon la revendication 8, caractérisé en ce qu'on solidifie la suspension homogène par une opération physique telle que dessiccation, évaporation, lyophilisation ou atomisation.

11. Utilisation d'une composition préparée conformément au procédé selon l'une quelconque des revendications 6 à 10, pour la préparation d'un médicament.

12. Utilisation des suspensions homogènes intégrales de plantes obtenues au moyen du procédé selon l'une quelconque des revendications 1 à 5, pour la préparation d'un médicament.

13. Application de la composition préparée selon l'une quelconque des revendications 6 à 10 et des suspensions homogènes obtenues au moyen du procédé selon l'une quelconque des revendications 1 à 5, à l'usage cosmétologique ou alimentaire.

**Patentansprüche**

**Patentansprüche für folgende Vertragsstaaten : AT, BE, CH, DE, DK, FR, GB, IT, LI, LU, NL, SE**

1. Verfahren zur Herstellung von homogenen Suspensionen frischer oder getrockneter Pflanzen, umfassend die Schritte: sorgfältiges Reinigen der Pflanzen oder Pflanzenteile und/oder Trocknen und Gefriervermahlen bei einer Temperatur von weniger als 0°C bis zum Erhalt einer Körnung von weniger als etwa 100 µm, dadurch gekennzeichnet, daß

1) nach Einweichen der Teilchen über einen Zeitraum von einigen Stunden bis mehr als 72 Stunden in mindestens einem Lösungsmittel, das geeignet ist zur Extraktion des (der) Wirkstoffs(e) der zu behandelnden Pflanze oder Pflanzenteile, der (die) in dem (den) Lösungsmittel(n) löslich ist (sind), die flüssige Phase von der festen Phase der erhaltenen Suspension in geeigneter Weise getrennt wird,
2) die feste Phase unter Druck in der Kälte ausgepreßt wird, und dann
3) die in 1) erhaltene flüssige Phase und die in 2) erhaltene herausgepreßte Flüssigkeit vermischt wird und eine homogene und vollständige Suspension der Pflanzen und/oder Pflanzenteile erhalten wird.

2.  Verfahren nach Anspruch 1, dadurch gekennzeichnet, daß das Einweichen im Schritt 1) mit Hilfe mindestens eines Lösungsmittels ausgewählt aus der Gruppe bestehend aus Wasser, Alkoholen, wie Ethanol, Polyalkoholen, wie Propylenglykol, Glycerin, Sorbit, Zuckeralkoholen, Maltodextrinen, Cyclodextrinen, Fettsäureestern, wie Isopropylmyristat, Fetten, wie Mineral-, Pflanzen- oder tierische Öle, und Tensiden durchgeführt wird.

3.  Verfahren nach Anspruch 2, dadurch gekennzeichnet, daß das oder die Lösungsmittel mindestens im Verhältnis 1:1 zum Gewicht der zu behandelnden Pflanze eingesetzt wird.

4.  Verfahren nach einem der Ansprüche 1 bis 3, dadurch gekennzeichnet, daß der im Schritt 2) ausgeübte Druck von der zu behandelnden Pflanze oder Pflanzenteil abhängig ist und vorzugsweise zwischen 1 und 100 bar liegt.

5.  Verfahren nach einem der Ansprüche 1 bis 4, dadurch gekennzeichnet, daß das Auspressen mit Hilfe einer Presse unter radialem Druck durchgeführt wird, wobei die feste Phase in dünnen Schichten verteilt wird.

6.  Flüssige Zusammensetzung, dadurch gekennzeichnet, daß sie eine homogene und vollständige Suspension von Pflanzen, die gemäß dem Verfahren nach einem der Ansprüche 1 bis 5 erhalten wurde, mindestens einen Aromastoff, mindestens ein Süßmittel und mindestens ein thixotropes Mittel umfaßt.

7.  Flüssige Zusammensetzung nach Anspruch 6, dadurch gekennzeichnet, daß sie außerdem mindestens eines der folgenden Mittel umfaßt: Verdickungsmittel, Duftstoffe, Gelbildner, appetitanregende Mittel.

8.  Feste Zusammensetzung, dadurch gekennzeichnet, daß sie eine homogene und vollständige Suspension von Pflanzen, die gemäß dem Verfahren nach einem der Ansprüche 1 bis 5 erhalten wurde, in fester Form umfaßt.

9.  Zusammensetzung nach Anspruch 8, dadurch gekennzeichnet, daß die homogene und vollständige Suspension von Pflanzen  auf einem geeigneten festen Absorptionsträger absorbiert ist.

10.  Zusammensetzung nach Anspruch 8, dadurch gekennzeichnet, daß die homogene Suspension durch ein physikalisches Verfahren, wie z.B. Trocknen, Eindampfen, Gefriertrocknen oder Spütrocknen, verfestigt wird.

11.  Verwendung der Zusammensetzung nach einem der Ansprüche 6 bis 10 für die Herstellung eines Medikaments.

12.  Verwendung der homogenen und vollständigen Suspensionen von Pflanzen, die gemäß dem Verfahren nach einem der Ansprüche 1 bis 5 erhalten wurden, zur Herstellung eines Medikaments.

13.  Verwendung der Zusammensetzung nach einem der Ansprüche 6 bis 10 und der homogenen Suspensionen, die gemäß dem Verfahren nach einem der Ansprüche 1 bis 5 erhalten wurden, als Kosmetika oder Lebensmittel.

**Patentansprüche für folgende Vertragsstaaten : ES; GR**

1.  Verfahren zur Herstellung von homogenen Suspensionen frischer oder getrockneter Pflanzen, umfassend die Schritte: sorgfältiges Reinigen der Pflanzen oder Pflanzenteile und/oder Trocknen und Gefriervermahlen bei einer Temperatur von weniger als 0°C bis zum Erhalt einer Körnung von weniger als etwa 100 μm, dadurch gekennzeichnet, daß
    1) nach Einweichen der Teilchen über einen Zeitraum von einigen Stunden bis mehr als 72 Stunden in mindestens einem Lösungsmittel, das geeignet ist zur Extraktion des (der) Wirkstoffs(e) der zu behandelnden Pflanze oder Pflanzenteile, der (die) in dem (den) Lösungsmittel(n) löslich ist (sind), die flüssige Phase von der festen Phase der erhaltenen Suspension in geeigneter Weise getrennt wird,

2) die feste Phase unter Druck in der Kälte ausgepreßt wird, und dann

3) die in 1) erhaltene flüssige Phase und die in 2) erhaltene herausgepreßte Flüssigkeit vermischt wird und eine homogene und vollständige Suspension der Pflanzen und/oder Pflanzenteile erhalten wird.

2. Verfahren nach Anspruch 1, dadurch gekennzeichnet, daß das Einweichen im Schritt 1) mit Hilfe mindestens eines Lösungsmittels ausgewählt aus der Gruppe bestehend aus Wasser, Alkoholen, wie Ethanol, Polyalkoholen, wie Propylenglykol, Glycerin, Sorbit, Zuckeralkoholen, Maltodextrinen, Cyclodextrinen, Fettsäureestern, wie Isopropylmyristat, Fetten, wie Mineral-, Pflanzen- oder tierische Öle, und Tensiden durchgeführt wird.

3. Verfahren nach Anspruch 2, dadurch gekennzeichnet, daß das oder die Lösungsmittel mindestens im Verhältnis 1:1 zum Gewicht der zu behandelnden Pflanze eingesetzt wird.

4. Verfahren nach einem der Ansprüche 1 bis 3, dadurch gekennzeichnet, daß der im Schritt 2) ausgeübte Druck von der zu behandelnden Pflanze oder Pflanzenteil abhängig ist und vorzugsweise zwischen 1 und 100 bar liegt.

5. Verfahren nach einem der Ansprüche 1 bis 4, dadurch gekennzeichnet, daß das Auspressen mit Hilfe einer Presse unter radialem Druck durchgeführt wird, wobei die feste Phase in dünnen Schichten verteilt wird.

6. Verfahren zur Herstellung einer flüssigen Zusammensetzung, dadurch gekennzeichnet, daß man eine homogene und vollständige Suspension von Pflanzen, die gemäß dem Verfahren nach einem der Ansprüche 1 bis 5 erhalten wurde, mit mindestens einem Aromastoff, mindestens einem Süßmittel und mindestens einem thixotropen Mittel mischt.

7. Verfahren nach Anspruch 6, dadurch gekennzeichnet, daß man außerdem mindestens eines der folgenden Mittel zufügt: Verdickungsmittel, Duftstoffe, Gelbildner, appetitanregende Mittel.

8. Verfahren zur Herstellung einer festen Zusammensetzung, dadurch gekennzeichnet, daß man eine homogene und vollständige Suspension von Pflanzen, die gemäß dem Verfahren nach einem der Ansprüche 1 bis 5 erhalten wurde, in eine feste Form bringt.

9. Herstellungsverfahren nach Anspruch 8, dadurch gekennzeichnet, daß man die homogene und vollständige Suspension von Pflanzen auf einem geeigneten festen Absorptionsträger absorbiert.

10. Herstellungsverfahren nach Anspruch 8, dadurch gekennzeichnet, daß man die homogene Suspension durch ein physikalisches Verfahren, wie z.B. Trocknen, Eindampfen, Gefriertrocknen oder Sprühtrocknen, verfestigt.

11. Verwendung einer gemäß dem Verfahren nach einem der Ansprüche 6 bis 10 hergestellten Zusammensetzung für die Herstellung eines Medikaments.

12. Verwendung der homogenen und vollständigen Suspensionen von Pflanzen, die gemäß dem Verfahren nach einem der Ansprüche 1 bis 5 erhalten wurden, zur Herstellung eines Medikaments.

13. Anwendung der gemäß dem Verfahren nach einem der Ansprüche 6 bis 10 hergestellten Zusammensetzung und der gemäß dem Verfahren nach einem der Ansprüche 1 bis 5 erhaltenen homogenen Suspensionen als Kosmetika oder Lebensmittel.

## Claims

### Claims for the following Contracting States : AT, BE, CH, DE, DK, FR, GB, IT, LI, LU, NL, SE

1. Process for the preparation of homogeneous suspensions of fresh or dried plants, comprising the steps of thorough cleaning of the plant or of plant part and/or of drying, and of cryocomminuting at a temperature below 0°C until a particle size of less than approximately 100 μm is obtained, which process is characterized:

1) in that after soaking particles for from a few hours to more than 72 hours in at least one solvent suitable for extracting the active ingredient(s) of the plant or plant part to be processed, which are soluble in the said solvent(s), the liquid phase and the solid phase of the suspension obtained are subjected to phase separation;
2) in that the solid phase is pressed out by cold pressing;
3) then in that the liquid phase obtained in 1) and the pressing obtained in 2) are mixed and form a homogeneous and perfect suspension of plants and/or plant parts.

2. Process according to Claim 1, characterized in that the soaking of step 1) is carried out with the aid of at least one solvent selected from the group comprising water, alcohols such as ethanol, polyalcohols such as propyleneglycol, glycerol, sorbitol, sugar alcohols, maltodextrins, cyclodextrins, fatty acid esters such as isopropyl myristate, fatty substances such as mineral, vegetable or animal oils, or surfactant agents.

3. Process according to Claim 2, characterized in that the solvent(s) are in a ratio of at least 1:1 in relation to the weight of the plant to be processed.

4. Process according to any of Claims 1 to 3, characterized in that the pressure exerted during step 2) depends on the plant or plant part to be processed and is preferably between 1 and 100 bar.

5. Process according to any of Claims 1 to 4, characterized in that pressing is carried out with the aid of a radial compression press, the solid phase being divided into fine layers.

6. Liquid composition, characterized in that it comprises a integral homogeneous suspension of plants obtained by means of the process according to any of Claims 1 to 5, at least one flavouring, at least one sweetener, and at least one thixotropic agent.

7. Liquid composition according to Claim 6, characterized in that it further comprises at least one of the following agents: thickeners, perfumes, gelling agents, appetizing agents.

8. Solid composition, characterized in that it comprises a integral suspension of plant obtained by means of the process according to any of Claims 1 to 5, put under a solid form.

9. Composition according to Claim 8, characterized in that the integral homogeneous suspension of plant is absorbed on an appropriate absorbent solid carrier.

10. Composition according to Claim 8, characterized in that the homogeneous suspension is solidified by a physical operation such as desiccation, evaporation, freeze drying or atomization.

11. Use of the composition according to any of Claims 6 to 10, for the preparation of a medicament.

12. Use of the integral homogeneous suspensions of plants obtained by means of the process according to any of Claims 1 to 5, for the preparation of a medicament.

13. Application of the composition according to any of Claims 6 to 10 and of the homogeneous suspensions obtained by means of the process according to any of Claims 1 to 5, for cosmetological or nutritional use.

**Claims for the following Contracting States : ES, GR**

1. Process for the preparation of homogeneous suspensions of fresh or dried plants, comprising the steps of thorough cleaning of the plant or of plant part and/or of drying, and of cryocomminuting at a temperature below 0°C until a particle size of less than approximately 100 μm is obtained, which process is characterized:
1) in that after soaking particles for from a few hours to more than 72 hours in at least one solvent suitable for extracting the active ingredient(s) of the plant or plant part to be processed, which are soluble in the said solvent(s), the liquid phase and the solid phase of the suspension obtained are subjected to phase separation;
2) in that the solid phase is pressed out by cold pressing;
3) then in that the liquid phase obtained in 1) and the pressing obtained in 2) are mixed and form a homogeneous and perfect suspension of plants and/or plant parts.

2. Process according to Claim 1, characterized in that the soaking of step 1) is carried out with the aid of at least one solvent selected from the group comprising water, alcohols such as ethanol, polyalcohols such as propyleneglycol, glycerol, sorbitol, sugar alcohols, maltodextrins, cyclodextrins, fatty acid esters such as isopropyl myristate, fatty substances such as mineral, vegetable or animal oils or surfactant agents.

3. Process according to Claim 2, characterized in that the solvent(s) are in a ratio of at least 1:1 in relation to the weight of the plant to be processed.

4. Process according to any of Claims 1 to 3, characterized in that the pressure exerted during step 2) depends on the plant or plant part to be processed and is preferably between 1 and 100 bar.

5. Process according to any of Claims 1 to 4, characterized in that pressing is carried out with the aid of a radial compression press, the solid phase being divided into fine layers.

6. Process for preparing a liquid composition, characterized in that a integral homogeneous suspension of plants obtained by means of the process according to any of Claims 1 to 5, at least one flavouring, at least one sweetener, and at least one thixotropic agent are mixed.

7. Process according to Claim 6, characterized in that at least one of the following agents are further added: thickeners, perfumes, gelling agents, appetizing agents.

8. Process for preparing a solid composition, characterized in that a integral suspension of plant obtained by means of the process according to any of Claims 1 to 5 is put under a solid form.

9. Preparation process according to Claim 8, characterized in that the integral homogeneous suspension of plant is absorbed on an appropriate absorbent solid carrier.

10. Preparation process according to Claim 8, characterized in that the homogeneous suspension is solidified by a physical operation such as desiccation, evaporation, freeze drying or atomization.

11. Use of a composition prepared in accordance with the process according to any of Claims 6 to 10, for the preparation of a medicament.

12. Use of the integral homogeneous suspensions of plants obtained by means of the process according to any of Claims 1 to 5, for the preparation of a medicament.

13. Application of the composition prepared according to any of Claims 6 to 10 and of the homogeneous suspensions obtained by means of the process according to any of Claims 1 to 5, for cosmetological or nutritional use.

FIGURE 1

Profil du pressat

Profil du surnageant

FIGURE 2

Profil du surnageant

Profil du pressat

FIGURE 3

Profil du pressat

Profil du surnageant

FIGURE 4